# EUROPEAN PATENT APPLICATION

(11) **EP 1 602 653 A1**
(43) Date of publication of application: **07.12.2005**
(21) Application number: 04719137.4
(22) Date of filing: 10.03.2004
(51) Int. Cl.: C07D 311/62, A61K 35/78

(54) **PROCESS FOR PRODUCING PROANTHOCYANIN-RICH MATERIAL**

(30) Priority: 11.03.2003 WO PCT/JP03/02877; 11.06.2003 WO PCT/JP03/07446; 06.08.2003 WO PCT/JP03/10032
(71) Applicant: Toyo Shinyaku Co., Ltd., Fukuoka-shi, Fukuoka 812-0011 (JP)
(72) Inventor: TAKAGAKI, Kinya, c/o Toyo Shinyaku Co., Ltd, Fukuoka-shi, Fukuoka 812-0011 (JP); MITSUI, Takeshi, c/o Toyo Shinyaku Co., Ltd, Fukuoka-shi, Fukuoka 812-0011 (JP); YAMAGUCHI, Gotaro, c/o Toyo Shinyaku Co., Ltd, Fukuoka-shi, Fukuoka 812-0011 (JP)
(74) Representative: Arth, Hans-Lothar
(86) International application number: PCT/JP2004/003158
(87) International publication number: WO 2004/080995

(57) **Abstract**

The method of the present invention, comprises the process of treating an extract or squeezed juice of a plant with a salt and/or an alkaloid and then with a synthetic resin adsorbent, or the process of treating an extract or squeezed juice of a plant with a synthetic resin adsorbent and then with a salt and/or an alkaloid. By this method, a proanthocyanidin-containing product containing highly bioactive OPCs at a high ratio can be obtained easily and efficiently.

## Description

### Technical Field

The present invention relates to a method for producing efficiently a proanthocyanidin-containing product containing a large amount of highly bioactive OPCs.

### Background Art

Proanthocyanidins are tannins that are present in various plants. These tannins are a group of compounds that are condensation or polymerization products (hereinafter, referred to as "polycondensation products") and have flavan-3-ol and/or flavan-3,4-diol as a constituent unit. When these compounds are subjected to acid treatment, anthocyanidins such as cyanidin, delphinidin, and pelargonidin are produced. Therefore, these compounds are designated as proanthocyanidins.

Proanthocyanidins, which are one type of polyphenol, are potent antioxidants produced by plants, and are contained concentratedly in portions of plant leaves, bark, or skin or seeds of fruits. More specifically, they are contained in the seeds of grape; the bark of pine; the inner skin of peanuts; the leaves of ginkgo; the fruit of locust; and cowberry, for example. Moreover, it is known that proanthocyanidins are also contained in cola nuts in West Africa; the roots of Rathania in Peru; and Japanese green tea. Proanthocyanidins cannot be produced in the human body.

Proanthocyanidins generally can be obtained by extraction from plants. Examples of solvent used for the extraction include water; organic solvents such as methanol, ethanol, acetone, hexane, ethyl acetate; or mixtures of these solvents (Japanese Laid-Open Patent Publication No. 11-80148). However, only extraction with a solvent provides a small amount of proanthocyanidin and purity of the resultant extract is low. Therefore, in order to use the extract for raw materials of health food products, cosmetics or pharmaceuticals, it is necessary to increase the purity. Thus, additional processes such as concentration and purification should be required, which increases the cost and time.

Methods for recovering polyphenols containing proanthocyanidins have been reported. For example, Japanese Laid-Open Patent Publication Nos. 5-279264 and 6-56689 describe a process of adsorbing polyphenols to a chitin substrate and the chitin substrate to which the polyphenols are adsorbed is utilized as a polyphenol product. Japanese Laid-Open Patent Publication No. 2002-97187 describes a method for recovering free polyphenols that comprises adding ascorbic acid and an alkali metal or a salt thereof to a plant extract liquid in order to adjust the pH in the range of 6 to 11, thereby precipitating a metal salt of the polyphenol, and removing this precipitate with an ion-exchange resin or the like.

In recent years, it has been reported that among the proanthocyanidins, condensation products having a lower degree of polymerization, in particular, condensation products having a degree of polymerization of 2 to 4 (dimer to tetramer) have an excellent antioxidation ability. In this specification, the condensation products having a degree of polymerization of 2 to 4 are referred to as "oligomeric proanthocyanidins" or "OPCs". In addition to the antioxidation ability, OPCs are also known to provide, for example, an effect of inhibiting bacterial proliferation in the oral cavity to reduce plaque (dental plaque); an effect of recovering the elasticity of blood vessels; an effect of preventing lipoprotein in blood from being damaged by active oxygen, thereby preventing aggregation and adherence of the oxidized fats onto the inside wall of the vessel, thus preventing cholesterol from being aggregated and adhered onto the oxidized fats that have been adhered onto the inside wall of the vessel; an effect of regenerating vitamin E that has been degraded by active oxygen; and an effect of serving as an enhancer of vitamin E.

However, most of the proanthocyanidins that are obtained by the above-described methods have a high degree of polymerization, and the content of highly bioactive OPCs (i.e., condensation products having a degree of polymerization of 2 to 4) is very low.

Japanese Laid-Open Patent Publication Nos. 4-190774, 10-218769, and 2001-131027 and "Proanthocyanidins from Krameria triandra Root" by Eberhard Scholz et al, Planta Medica, 55(1989), pp. 379 to 384 describe methods for extracting OPCs from plants and/or synthesizing OPCs. In the extraction method, an extract liquid of a plant is brought into contact with an adsorbent, the adsorbed material is eluted, and specific fractions are collected, and thereafter the same process is repeated using the collected fractions. Without repeating the process, the content of the OPCs cannot be increased, which is not efficient. The synthesizing method includes a lot of steps, which causes problems of high cost and long time, and also causes problems of liquid waste disposal.

Therefore, there is a demand for a method for producing proanthocyanidins containing a large amount of OPCs.

### Disclosure of Invention

The inventors of the present invention conducted in-depth studies on a method for efficiently obtaining a proanthocyanidin-containing product that contains useful highly bioactive OPCs at a high ratio. As a result, the present invention was achieved by finding out that a proanthocyanidin-containing product that contains highly bioactive OPCs at a high ratio can be obtained easily and efficiently by the process of treating an extract or squeezed juice of a plant with a salt and/or an alkaloid and then with a synthetic resin adsorbent; or the process of treating an extract or squeezed juice of a plant with a synthetic resin adsorbent and then with a salt and/or an alkaloid.

A method of the present invention for producing a proanthocyanindin-containing product obtained from an extract or squeezed juice of a plant comprises, subjecting an extract or squeezed juice of a plant to a combination of the following treatments: a treatment with at least one of a salt and an alkaloid, and a treatment with a synthetic resin adsorbent.

In a preferred embodiment, the method further comprises a concentration process that is conducted before and/or after the treatment with at least one of a salt and an alkaloid, wherein the concentration process excludes the treatment with a synthetic resin adsorbent.

A method of the present invention for producing a proanthocyanidin-containing product comprises the steps of: subjecting an extract or squeezed juice of a plant to a concentration process to obtain a concentrate; treating the concentrate with at least one of a salt and an alkaloid to obtain a crude proanthocyanidin-containing product; and treating the crude proanthocyanidin-containing product with a synthetic resin adsorbent.

In a preferred embodiment, the method further comprises a concentration process that is conducted before causing an insoluble substance by the treatment with at least one of a salt and an alkaloid and/or after removing the insoluble substance, wherein the concentration process excludes the treatment with a synthetic resin adsorbent.

In a preferred embodiment, the salt is at least one selected from the group consisting of a monovalent alkali metal salt, a divalent alkali metal salt, and ammonium sulfate.

In a preferred embodiment, the alkaloid is at least one selected from the group consisting of betaine, caffeine and their derivatives.

In a preferred embodiment, the the proanthocyanidin-containing product comprises at least 20 wt% of oligomeric proanthocyanidins in terms of dry weight.

The present invention also provides a proanthocyanidin-containing product that comprises at least 20 wt% of oligomeric proanthocyanidins in terms of dry weight.

In a preferred embodiment, the proanthocyanidin-containing product further comprises 5 to 15 wt% of catechins in terms of dry weight.

### Best Mode for Carrying Out the Invention

A method for producing a proanthocyanidin-containing product of the present invention comprises, the process of treating an extract or squeezed juice of a plant with a salt and/or an alkaloid, and then with a synthetic resin adsorbent, or the process of treating an extract or squeezed juice of a plant with a synthetic resin adsorbent, and then with a salt and/or an alkaloid. Preferably, a concentration is conducted before and/or after the treatment with a salt and/or alkaloid.

### (Extract or squeezed juice of a plant)

First, an extract or squeezed juice of a plant is obtained.

There is no limitation on the type of the plant used in the present invention, as long as the plant contains a proanthocyanidin. Examples of the plant include bark of Cryptomeria japonica, white cedar, pine, and the like; the fruit, skins of fruit or seeds of plants such as grape, blueberry, strawberry, avocado, locust, cowberry, and elderberry; barley; wheat; soybean; black soybean; cacao; adzuki bean; the hull of conker; the inner skin of peanuts; the leaves of ginkgo; tea leaves and tea extract liquid; sorghum; apple fruits; Sasa veitchii; fucoidan; yacon leaves; cola nuts (e.g., cola nuts in West Africa); and the roots of Rathania (e.g., Rathania in Peru). Among these, in particular, pine bark, grape seeds and fruit skin of grape, and the inner skin of peanuts are preferably used.

In order to obtain an extract of a plant, an extraction solvent is added to the plant, and the mixture is kept at a predetermined temperature as necessary.

When performing extraction, in view of extraction efficiency, it is preferable that a plant is pulverized to an appropriate size to increase the surface area per volume. There is no limitation on the method for pulverization. For example, pulverized products obtained by the use of a cutter, slicer, or the like; ground products obtained by the use of a mixer, a juicer, a blender, masscollider or the like can be employed. The pulverized product or the ground product is a small piece having a size of 0.1 to 10 cm, preferably 0.1 to 5 cm. In order to increase the pulverization efficiency, water or an organic solvent such as ethanol, methanol or ethyl acetate may be added at the time of pulverization.

As the extraction solvent, water or an organic solvent is used. Examples of the organic solvent include methanol, ethanol, 1-propanol, 2-propanol, 1-butanol, 2-butanol, acetone, hexane, cyclohexane, propylene glycol, aqueous ethanol, aqueous propylene glycol, methyl ethyl ketone, glycerin, methyl acetate, ethyl acetate, diethyl ether, dichloromethane, edible oils or fats, 1,1,1,2-tetrafluoroethane, and 1,1,2-trichloroethene. Furthermore, a mixed solvent of water and organic solvents may be preferably used. These organic solvents can be used alone or in combination. In view of the disposal of liquid waste formed in the production process, or in view of the treatment of adding a salt and/or an alkaloid or the treatment with a synthetic resin adsorbent described later, water or ethanol are preferably used.

In view of the fact that concentration should be performed at a relatively low temperature for a short period of time, an organic solvent (e.g., ethanol) having a lower boiling point than that of water, or a mixed solvent of the organic solvent and water is preferably used as the extraction solvent among the above-described extraction solvents. Such organic solvent can be removed easily at the time of the concentration. Among these, in view of safety when used as foods or pharmaceuticals, ethanol or a mixed solvent of ethanol and water is particularly preferable.

The amount of the extraction solvent that is added to a plant can be determined in view of the desired concentration of proanthocyanidins and the extraction efficiency. For example, when water is used as an extraction solvent, the weight ratio of the plant and water is 1:5 to 1:100, preferably 1:10 to 1:50. When water and/or an organic solvent is added and then pulverization is performed, the amount of the extraction solvent to be added can be determined by taking the amount of water and/or the organic solvent used for the pulverization into account.

A higher extraction temperature is preferable in order to increase the extraction efficiency. For example, when water is used, hot water with 50 °C to 120 °C, preferably 70 °C to 100 °C is used for extraction. Hot water may be added to the plant, and after water is added to the plant, the mixture can be heated. The extraction time is determined as appropriate, depending on the extraction temperature. The extraction time is generally 10 minutes to 24 hours.

As the method for extraction with an organic solvent, a heat extraction method or a supercritical fluid extraction method is preferable. As the heat extraction method, a process of adding a warmed solvent to a plant, or a process of adding a solvent to a plant and then heating the resultant mixture can be employed. For example, a water-ethanol mixed solvent comprising water and ethanol at a weight ratio of 1:1 to 1:9 is used as the extraction solvent in an amount of 1 to 20 times the amount of the pulverized plant. Then, the extraction is performed by stirring the resultant mixture for 0.5 to 6 hours while being refluxed at 70 °C to 75 °C. When the temperature of the extraction is not raised to the temperature of reflux, the extraction efficiency can be increased by the process including heat extraction once with the mixed solvent, recovering of the supernatant from the resultant mixture by filtration, further addition of the mixed solvent to the residue, and warming the resultant mixture.

Supercritical fluid extraction is a method for extracting a target component, using a supercritical fluid. A supercritical fluid is in a state that is above the liquid-vapor critical point in the phase diagram showing critical temperature and critical pressure. Examples of compounds that can be employed as a supercritical fluid include carbon dioxide, ethylene, propane, and nitrous oxide (laughter gas). Carbon dioxide is preferably used.

Supercritical fluid extraction includes an extraction step in which a target component is extracted with a supercritical fluid and a separation step in which the target component is separated from the supercritical fluid. In the separation step, any separation process can be employed, examples of which include a separation based on a change in pressure, a separation based on a change in temperature, and a separation using an adsorbent or absorbent.

Moreover, it is also possible to perform supercritical fluid extraction in which an entrainer is added. In this method, extraction is performed using an extracting fluid obtained by adding, for example, ethanol, propanol, n-hexane, acetone, toluene, or another aliphatic lower alcohol, aliphatic hydrocarbon, aromatic hydrocarbon, or ketone at about 2 to 20 w/v% to a supercritical fluid, so that the solubility of a target substance to be extracted, such as OPCs and catechins (described later), in the extracting fluid is dramatically increased or the selectivity of separation is enhanced. Thus, proanthocyanidins are obtained efficiently.

For extraction, any of apparatuses, for example, batch type, semi-continuous, or continuous extraction apparatuses can be used.

In order to obtain squeezed juice of the plant, the plant is directly squeezed, or cut or pulverized as appropriate and then squeezed. This method is employed preferably when a plant having a high moisture content is used. For example, in the case of grape fruits, squeezed juice containing proanthocyanidins can be obtained by squeezing. Plant pulverized products (e.g., pulverized products of fruits of grape) containing a solid content derived from a plant obtained by pulverizing the plant can be used as well as the squeezed juice. In this specification, the plant pulverized products containing a solid content derived from the plant are included in the squeezed juice.

### (Concentration process)

It is preferable that the extract or squeezed juice (hereinafter, referred to as "extract or the like") of the plant is concentrated in advance before a treatment with a salt and/or alkaloid. By concentrating the extract or the like of the plant, the treatment with a salt and/or alkaloid can be performed with a small amount of the salt or alkaloid, and furthermore, proanthocyanidins having a high degree of polymerization can be removed efficiently. When performing the concentration, it is preferable that filtration is conducted in advance to remove insoluble substances in the extract or the like, because this makes it possible to perform concentration uniformly and makes it easy to control the concentration degree of the resultant concentrate.

As the method for concentration, the method that are commonly used by those skilled in the art can be employed. Examples of the method include heating concentration, vacuum concentration, freeze drying, concentration by treatment with a synthetic resin adsorbent, concentration with an ultrafiltration membrane, concentration with a dialysis membrane and the like. In view of thermal denaturation of proanthocyanidins, vacuum concentration, freeze drying, and concentration by treatment with a synthetic resin adsorbent are preferable, and vacuum concentration and concentration by treatment with a synthetic resin adsorbent are more preferable. These concentration methods can be employed alone or in combination with a plurality of methods.

In the case of conducting the heating concentration, heating is performed at a temperature of 40°C to 100°C in order to prevent the thermal denaturation of proanthocyanidins.

The concentration by treatment with a synthetic resin adsorbent is conducted by a process of performing an adsorption treatment with a synthetic resin adsorbent that can adsorb preferentially OPCs, which will be described later, and eluting the adsorbed OPCs by the use of a solvent so as to obtain a solution (concentrate) containing OPCs at a concentration higher than the initial extract or the like. In the process of concentration by treatment with a synthetic resin adsorbent, an extraction solvent that has been used for extraction can be substituted with an organic solvent having a high volatility that can facilitate concentration. By the employment of such organic solvent, it is possible to obtain a concentrate having a higher concentration degree easily by vacuum concentration compared with the case of employing water as the extraction solvent. For example, when water (e.g., hot water) is used as the extraction solvent, an organic solvent (e.g., ethanol) or a mixed solvent of an organic solvent and water is used for eluting OPCs from the adsorbent. Then, concentration process can be conducted simply by removing the organic solvent to a necessary extent or removing the organic solvent completely, and then adding an appropriate solvent in a necessary amount. In particular, in the case of employing an aromatic resin such as DIAION HP-20, which will be described later, concentration of proanthocyanidins with a synthetic resin adsorbent is simply performed by adsorbing proanthocyanidins or the like to the synthetic resin adsorbent, and then recovering all the adsorbed substances from the column with absolute ethanol. Furthermore, the ethanol in the concentrate can be removed by vacuum concentration in a simple manner, so that a concentrate having a higher concentration degree can be obtained.

There is no limitation on the concentration degree of the resultant concentrate. The concentration is performed such that the volume of the concentrate is preferably 1/2 to 1/100, more preferably 1/5 to 1/70, even more preferably 1/10 to 1/50 of the volume of the extract or the like before the concentration.

The concentration process can be performed, not only before the treatment with a salt and/or alkaloid, but also performed whenever necessary, for the purpose of removing an undesired solvent for any treatment. Preferably, concentration is performed before or after the treatment with a salt and/or alkaloid; more preferably before the treatment with a salt and/or alkaloid.

When a substance that is to be treated (e.q., an extract or the like) contains an organic solvent, the organic solvent can be removed and substituted with water in the concentration (dialysis, vacuum concentration or the like). By substituting the organic solvent with water; ionization of the salt and/or the alkaloid in the solution is improved, so that proanthocyanidins having a high degree of polymerization can be insolubilized efficiently. When substitution with the water is carried out, the final volume is preferably less than twice the original volume of the extract or the like.

### (Salt and/or alkaloid treatment process)

This process can comprise adding a salt and/or an alkaloid to an extract or squeezed juice of the plant, a concentrate thereof, or a synthetic resin adsorbent-treated product of any one of these materials (i.e., a synthetic resin adsorbent-treated liquid). By adding a salt and/or an alkaloid to the extract or the like, concentrate thereof or the synthetic resin adsorbent-treated liquid, the proanthocyanidins having a high degree of polymerization deposit as an insoluble substance such as a precipitate, or the proanthocyanidins having a high degree of polymerization are converted so as to have properties with which the proanthocyanidins are hardly adsorbed to the synthetic resin adsorbent (hereinafter, the resultant proanthocyanidins are referred to as "non-adsorptive proanthocyanidins"). Such insoluble substance can be removed by, for example, filtration, easily, and the non-adsorptive proanthocyanidins can be removed in a subsequent synthesis resin adsorbent treatment. Thus, proanthocyanidins with a high content of OPCs can be obtained. The salt and/or alkaloid can be used alone or in combination.

The salt used in the present invention may be any salt, as long as it can ionize in a solution. Examples of the salt include monovalent metal salt, divalent metal salts, and nonmetallic salts. Monovalent metal salts and nonmetallic salts are particularly preferable in that they can allow proanthocyanidins having a high degree of polymerization to deposit as an insoluble substance. Divalent metal salts are also particularly preferable in that they can convert proanthocyanidins having a high degree of polymerization into non-adsorptive proanthocyanidins.

Examples of the monovalent metal salts include salts of alkali metals such as lithium, sodium, potassium, rubidium, cesium and francium. For example, alkali metal halides (alkali metal chlorides, alkali metal bromides, etc.), alkali metal phosphate, alkali metal carbonate, and alkali metal organic acid salts (carboxylate such as acetate, sulfonate, etc.) are included. Specific examples of the monovalent metal salts include sodium chloride, sodium sulfate, sodium citrate, potassium chloride, sodium phosphate, potassium phosphate, and sodium acetate. In particular, sodium sulfate, potassium phosphate, sodium citrate and sodium chloride, which can be used preferably for salting-out, are preferable.

Examples of the divalent metal salts include salts of metals, the metals being beryllium, magnesium, alkaline-earth metals (e.g., calcium, strontium, barium, radium, and the like). Magnesium salts and calcium salts are preferable, in particular, magnesium sulfate is more preferable. When divalent metal salts containing a metal (e.g., copper) that can be employed as an antioxidant is used, proanthocyanidins may be oxidized, so that care should be taken.

The divalent metal salts especially have a high adsorption ability to proanthocyanidins, so that proanthocyanidins having a high degree of polymerization can be converted to insoluble substances or non-adsorptive proanthocyanidins even in a small amount of the salt.

In the treatment with the metal salt, it is preferable to perform the treatment in an acidic condition. When proanthocyanidins are treated in a condition of weak to strong alkalinity, the stability of the proanthocyanidins is poor so that the proanthocyanidins may be decomposed. For this reason, the pH of the solution is adjusted preferably to lower than 7.5, more preferably lower than 6, even more preferably 5.5 or lower. In particular, when the divalent metal salt is used, care should be taken because the pH in the solution easily increases by the addition of the salt. The pH can be adjusted, using, for example, an aid (pH regulator such as ascorbic acid) for stabilizing proanthocyanidins, water of a low pH (low pH water) that has been treated with cation exchange resin, or the like. In particular, the low pH water is preferable in view of the overall cost and the fact that a removal process is not necessary, compared with the case where a pH regular is added. More specifically, when the low pH water is employed, the treatment with the salt is conducted in the following manner. First, a metal salt solution is prepared by adding a metal salt to low pH water such that the concentration of the metal salt is 2 to 10 times the final concentration at the time of salt treatment. This solution has a pH of 4 to 6, preferably, 4 to 5.5, more preferably 4 to 5. Then, this solution is added to the extract or the like for treatment, se that decomposition of the proanthocyanidins can be prevented.

Preferable example of non-metallic salt include ammonium sulfate.

Among the above-described salts, in order to precipitate proanthocyanidins having a high degree of polymerization selectively, and to increase the purification efficiency of OPCs, in particular, the monovalent alkali metal salts or ammonium sulfate are preferable. In order to reduce the amount of the salt used, the divalent metal salts are preferable, which increase the purification efficiency by converting the proanthocyanidins having a high degree of condensation into non-adsorptive proanthocyanidins. In view of the fact that the amount of the salt used is small, alkaloid, which will be describe later, is also preferable.

There is no limitation on the amount of the salt added, and the amount can be set as appropriate, depending on the type of salts. For example, the salt can be added in a 0.0001 weight/volume% to 50 weight/volume% in the extract or the like, the synthetic resin adsorbent-treated liquid, or the concentrate.

When the monovalent metal salt is employed, the salt can be added in the extract or the like, the synthetic resin adsorbent-treated liquid, or the concentrate preferably in an amount of 0.1 weight/volume% to 50 weight/volume%, more preferably 3 weight/volume% to 50 weight/volume%, even more preferably 5 weight/volume% to 45 weight/volume%, most preferably 8 weight/volume% to 45 weight/volume%. In particular, a salt is preferably added to the extract or the like, the synthetic resin adsorbent-treated liquid, or the concentrate such that it is contained in an amount of 10 to 75%, preferably 20 to 60% of a saturated concentration of the salt, when the maximum amount (saturated concentration) of the salt that can be dissolved in water with 25°C (e.g., 35.8 weight/volume% in the case of sodium chloride) is taken as 100%.

When the divalent metal salt such as calcium salt or magnesium salt is employed, the salt can be added to the extract or the like, the synthetic resin adsorbent-treated liquid, or the concentrate, so that the concentration of the metal salt is preferably in the range of 0.0001 weight/volume% to 30 weight/volume%, and more preferably 0.001 weight/volume% to 10 weight/volume%. Furthermore, it is preferable to add the divalent metal salt so that the concentration of the metal salt is preferably in the range of about 0.002 parts by weight to 4 parts by weight with respect to 100 parts by weight (dry weight) of the extract or the like of the plant (e.g., pine bark).

When the non-metallic salt is used, the salt can be added to the extract or the like, the synthetic resin adsorbent-treated liquid, or the concentrate, so that the concentration of the salt is preferably in the range of 8 weight/volume% or more, more preferably 8 weight/volume% to 80 weight/volume%.

The alkaloid used in the present invention is any of nitrogen-containing organic compounds that are generally classified into alkaloid. The alkaloid is excellent in that it converts proanthocyanidins having a high degree of polymerization into insoluble substances or non-adsorptive proanthocyanidins, in particular non-adsorptive proanthocyanidins.

Examples of the alkaloid include xanthine derivatives such as caffeine, derivative of simple amines (betaine, ete.), pyrrole alkaloid, pyrrolidine alkaloid, pyridine alkaloid, piperidine alkaloid, tropane alkaloid, imidazole alkaloid, purine alkaloid, quinoline alkaloid, isoquinoline alkaloid, quinazoline alkaloid, pyrolizidine alkaloid, quinolizidine alkaloid, indolizine alkaloid, phenanthrene alkaloid, erythrinan alkaloid, indole alkaloid, alkaloid including tropolone ring, diterpene alkaloid, steroid alkaloid, triterpene alkaloid, and colchicine. In the present invention, among these, derivative of simple amines or purine alkaloid are preferably employed, and especially, derivative of an amine such as betaine or a xanthine derivative such as caffeine is preferably used. Caffeine is particularly preferable because both the effect of converting proanthocyanidins having a high degree of polymerization into non-adsorptive proanthocyanidins and the effect of converting them into insoluble substances are excellent.

The alkaloid can be added to the extract or the like, the synthetic resin adsorbent-treated liquid, or the concentrate, so that the concentration of the alkaloid is preferably in the range of 0.00005 weight/volume% to 5 weight/volume%, more preferably 0.0001 weight/volume% to 3 weight/volume%, even more preferably 0.0001 weight/volume% to 1 weight/volume%. Furthermore, it is preferable to add the alkaloid so that the concentration of the alkaloid is preferably in the range of about 0.0002 parts by weight to 0.5 parts by weight with respect to 100 parts by weight (dry weight) of the extract or the like of the plant (e.g., pine bark).

When the concentrate of the extract or the like is used, the amount of the salt and/or the alkaloid used is preferably 1/2 to 1/100, more preferably 1/5 to 1/50 of the above-described amount.

There is no particular limitation on the temperature of the treatment with the salt and/or alkaloid. It is preferably 1°C to 40°C. There is also no particular limitation on the treatment time, and the treatment time can be set as appropriate, depending on the treatment temperature. For example, after the salt and/or the alkaloid is added to the extract or the like, the synthetic resin adsorbent-treated liquid, or the concentrate, are allowed stand at 1°C to 40°C for 30 minutes to 48 hours to deposit a sufficient amount of an insoluble substance such as a precipitate. The time for standing may be more than 48 hours, but preferably, the resultant mixture is preferable to transfer to the next step before OPCs are automatically oxidized and thus, change its color from reddish-brown to dark blackish-brown.

Then, the resultant insoluble substance such as a precipitate can be removed. By removing the insoluble substance, the following synthetic resin adsorbent treatment can be performed in a short period of time. As a method for removing the insoluble substance, any of the methods commonly used by those skilled in the art, for example, filtration or centrifugation, can be employed. In view of the treatment time, filtration can be preferably used. Filtration can be performed preferably at 1°C to 40°C. The lower the temperature of filtration is, the more proanthocyanidins having a high degree of polymerization can be removed. Filtration can be performed more preferably at 30°C or less, more preferably 25°C or less. In order to minimize the loss of proanthocyanidins due to filtration, the residue after filtration can be washed with the same solvent as used for the treatment with the salt and/or alkaloid, and the washed liquid may be combined. In the treatment with the salt and/or alkaloid, especially when the divalent metal salt or the alkaloid is used, proanthocyanidins having a high degree of polymerization are converted mostly into non-adsorptive proanthocyanidins, so that relatively small amount of insoluble substance is formed. When relatively small amount of insoluble substance is formed, the synthetic resin adsorbent treatment may be performed without removing the insoluble substance to reduce the cost.

Thus, a crude proanthocyanidin-containing product is obtained. The amount of the proanthocyanidines having a degree of polymerization of 5 or more contained in the supernatant of the product is 1/2 or less, preferably 1/3 or less, more preferably 1/5 or less, and even more preferably 1/6 or less of the amount of the concentrate before the treatment with the salt and/or alkaloid.

### (Process of treatment with the use of synthetic resin adsorbent)

This process is conducted with respect to the crude proanthocyanidin-containing product obtained by the treatment with the salt and/or alkaloid, or conducted with respect to the extract or squeezed juice of the plant or the concentrate before the treatment with the salt and/or alkaloid. By performing treatment with a synthetic resin adsorbent, contaminants such as saccharides and organic acids are removed. For example, when the extract or the like is treated with a synthetic resin adsorbent, contaminants as described above are removed, so that the following salt treatment can be performed efficiently. More specifically, the treatment with a synthetic resin adsorbent is performed in the following manner. First, the extract or the like, or the crude proanthocyanidin-containing product is brought into contact with a synthetic resin adsorbent so that the proanthocyanidins are adsorbed to the synthetic resin adsorbent and then are eluted with a predetermined solvent. When treating the extract or the like, or the crude proanthocyanidin-containing product with a synthetic resin adsorbent, it is preferable to remove the insoluble substances in advance in order to perform the treatment efficiently.

Examples of the synthetic resin adsorbent used in this treatment include organic resins, ion-exchange resins, silica gel, and silica gel used for reverse phase chromatography.

As the organic resins, aromatic resins such as a copolymer of styrene and divinylbenzene, an methacrylic acid type resin, or the like can be used. Aromatic resins are preferable. Example of aromatic resins include aromatic resins having a hydrophobic substituent, aromatic resins with no substituent, aromatic resins obtained by subjecting an aromatic resin with no substituent to a specific treatment. Aromatic resins obtained by subjecting an aromatic resin with no substituent to a specific treatment are preferable. It is preferable that these resins are porous. Such synthetic resins are commercially available, and examples thereof include the following: examples of methacrylic acid type resin include DIAION (registered trademark) HP1MG and DIAION HP2MG (manufactured by Mitsubishi Chemical Corporation); examples of aromatic resins include SP-900 (manufactured by Mitsubishi Chemical Corporation), Amberlite (registered trademark) XAD-2, Amberlite XAD-4, Amberlite XAD-16, and Amberlite XAD-2000 (manufactured by ORGANO CORPORATION); examples of aromatic resins having a hydrophobic substituent include DIAION (registered trademark) SP-205, DIAION SP-206, DIAION SP-207, DIAION HP-2MG, and DIAION EX-0021 (manufactured by Mitsubishi Chemical Corporation), Amberlite (registered trademark) XAD-7, and Amberlite XAD-8 (manufactured by ORGANO Corporation); examples of aromatic resins with no substituent are DIAION (registered trademark) HP-10, DIAION HP-20, DIAION HP-21, DIAION HP-30, DIAION HP-40, and DIAION HP-50 (manufactured by Mitsubishi Chemical Corporation); examples of aromatic resins obtained by subjecting an aromatic resin with no substituent to a specific treatment include SP-825, SP-800, SP-850, and SP-875 (manufactured by Mitsubishi Chemical Corporation); examples of crosslinked dextran derivatives include Sephadex (registered trademark) LH20, and Sephadex LH60 (manufactured by Pharmacia Biotech). Among these, aromatic resins (DIAION or Amberlite) or crosslinked dextran derivatives (Sephadex LH20, etc.) are preferable.

As ion exchange resins, both of cation exchange resin and anion exchange resin can be used. Examples of commercially available cation resins include Amberlite (registered trademark) CG-4000, Amberlite CG-5000, Amberlite CG-6000, Amberlite CG-8000, Amberlite IR-116, Amberlite IR-118, Amberlite IR-120B, Amberlite IR-122, Amberlite IR-124, Amberlite XT-1007, Amberlite XT-1009, and Amberlite XT-1002 (manufactured by ORGANO Corporation), which are resins having a sulfonate group as a functional group. Examples of weak basic anion exchange resins include OPTIPORE-XUS 40285.00 and OPTIPORE-XUS 40390.00 (manufactured by The Dow Chemical Company), which are resins having a quarternary ammonium group as a functional group. When using ion exchange resin is water, a preferable elution solvent and the column temperature is preferably 10°C to 120°C. The inside of the column is preferably at atmospheric pressure or pressurized.

The amount of the synthetic resin adsorbent can be determined as appropriate, depending on the type of the solvent, the type of the synthetic resin adsorbent and the like. For example, the synthetic resin adsorbent is preferably used in an amount that is 0.01 to 30 times, preferably 0.1 to 10 times the dry weight of a liquid to be treated. When the amount of the synthetic resin adsorbent is less than 0.01 times the dry weight of a liquid to be treated, the yield of the proanthocyanidins may be low.

When a synthetic resin adsorbent having a high efficiency of adsorbing proanthocyanidins, such as DIAION, which is an aromatic resin, Amberlite, which is an aromatic resin, or Sephadex, which is a crosslinked dextran derivative, is employed, then the operation can be performed in a simpler manner using such a resin in an amount based on the volume of the liquid to be treated, without taking the dry weight of the liquid to be treated into account. This is because the adsorbing efficiency of the resin is good. When the above-described synthetic resin adsorbent is 0.01 to 50 times, preferably 0.1 to 20 times the volume of the liquid to be treated is used, the liquid to be treated can be in sufficient contact with the synthetic resin adsorbent so that proanthocyanidins can be adsorbed efficiently, wherein the volume of the synthetic resin adsorbent is a volume swollen with water or an organic solvent.

The extract or the like or the crude proanthocyanidin-containing product can be brought into contact with the synthetic resin adsorbent by any method. For example, convenient methods such as a column chromatography method and a batch method can be employed. The column chromatography method comprises the steps of filling the synthetic resin adsorbent in a column and applying the extract or the like or the crude proanthocyanidin-containing product on the column. The batch method comprises the steps of adding the synthetic resin adsorbent to the extract or the like or the crude proanthocyanidin-containing product and removing the synthetic resin adsorbent after a predetermined period of time.

In order to carry out the column chromatography method, for example, first, the synthetic resin adsorbent is filled in a column, and the extract or the like or the crude proanthocyanidin-containing product is applied on the column. Then, water with a volume of 5 to 10 times the volume the synthetic resin adsorbent is passed through the column, thereby removing saccharides or organic acids, which are impurities. Thereafter, proanthocyanidins are eluted with an appropriate solvent. As the solvent, water, methanol, ethanol, ethyl acetate, chloroform, and mixed solvent of these can be used. A mixed solvent of water and ethanol is preferably used in view of safety. The mixing ratio of water and ethanol is varied depending on the kind of synthetic resin adsorbent. For example, in the case of an aromatic resin such as DIAION HP-20, an aqueous solution of ethanol with 5 to 50 volume%, preferably 10 to 40 volume%, more preferably 10 to 30 volume% can be used.

In order to carry out the batch method, the synthetic resin adsorbent with the same weight ratio as in the column chromatography method is added to the extract or the like or the crude proanthocyanidin-containing product and is brought into contact with it for one to three hours while stirring. Then, the adsorbent is recovered by filtration or centrifugation. The synthetic resin adsorbent to which proanthocyanidins are adsorbed is added to a solvent with the same composition as in the column chromatography method and stirred for one to three hours to release proanthocyanidins. Then the supernatant is obtained by filtration or centrifugation. Thus, a product treated with a synthetic resin adsorbent or a proanthocyanidin-containing product containing a larger amount of proanthocyanidins or OPCs can be obtained.

The yield of the proanthocyanidin-containing product obtained by the method of the present invention is 0.4 to 3 parts by weight, preferably 0.5 parts by weight to 2 parts by weight with respect to 100 parts by weight (dry weight) of the pine bark, when alkaloid or a divalent metal salt is used in the treatment with a salt and/or alkaloid.

### (Production process)

As described above, in the production method of the present invention, there is no limitation regarding the order of the treatment with the salt and/or alkaloid and the treatment with the synthetic resin adsorbent, and either process can be performed before the other. In other words, first, the extract or the like may be treated with the salt and/or the alkaloid, and then treated with the synthetic resin adsorbent, or the extract or the like may be treated with the synthetic resin adsorbent, and then treated with the salt and/or alkaloid. The former order is preferable because it is not necessary to provide treatment processes with an osmotic membrane or an adsorbent to remove the salt and/or the alkaloid in the solution, and the efficiency of treating with the synthetic resin adsorbent is increased, so that the content of OPCs can be increased. Furthermore, a concentration process or a process of removing the insoluble substance may be added before or after the treatment with the salt and/or alkaloid, if necessary.

The production method of the present invention comprises the following processes and is performed preferably in the following order: a process of subjecting an extract or squeezed juice of a plant to a concentration process to obtain a concentrate; a process of treating the concentrate with at least one of a salt and an alkaloid to obtain a crude proanthocyanidin-containing product; and a process of treating the crude proanthocyanidin-containing product with a synthetic resin adsorbent. More preferably, the concentration is performed by using a synthetic resin adsorbent. Namely, the method is conducted by a process of treating an extract or squeezed juice of a plant with a synthetic resin adsorbent to obtain a concentrate; the process of treating the concentrate with a salt and/or alkaloid to obtain a crude proanthocyanidin-containing product; and a process of treating the crude proanthocyanidin-containing product with a synthetic resin adsorbent. It should be noted that after these processes, a process of further purification (e.g., desalting process with an osmotic membrane or an adsorbent, etc.) may be added.

### (Proanthocyanidin-containing product)

The thus obtained proanthocyanidin-containing product contains proanthocyanidins at a high ratio. Herein, the proanthocyanidin-containing product includes a concentrate, a diluted product, powder and the like that can be thereafter obtained by a method commonly employed by those skilled in the art. For the proanthocyanidins in the product, proanthocyanidins having a low degree of condensation are preferable in view of bioactivities. As the condensation products having a low degree of polymerization, condensation products having a degree of polymerization of 2 to 30 (dimer to tridecamer) are preferable, condensation products having a degree of polymerization of 2 to 10 (dimer to decamer) are more preferable, and condensation products having a degree of polymerization of 2 to 4 (dimer to tetramer) are even more preferable. The proanthocyanidin-containing product obtained by the production method of the present invention contains a large amount of dimer to tetramer (oligomeric proanthocyanidins; OPC). The proanthocyanidin-containing product contains at least 20 wt%, more preferably, at least 30 wt%, more preferably at least 35 wt%, particularly preferably at least 50 wt% of OPCs in the product in terms of dry weight.

In particular, in the case where the method of the present invention comprises the concentration process before or after the treatment with the salt and/or alkaloid, the proanthocyanidin-containing product obtained by this method contains preferably at least 35 wt%, more preferably at least 40 wt%, even more preferably 40 wt% to 80 wt% of OPCs in the product in terms of dry weight. This OPC content of the proanthocyanidin-containing product is preferably at least twice, more preferably 2 to 4 times the content of OPCs in a treated product obtained by performing only the synthetic resin adsorbent treatment without subjecting the extract or the like to the concentration and the treatment with the salt and/or alkaloid. Furthermore, the OPC content of the proanthocyanidin-containing product is preferably at least 1.3 times, more preferably 1.3 to 2 times the content of OPCs in a treated product obtained without the concentration.

The ratio of the OPCs in the total proanthocyanidins contained in the proanthocyanidin-containing product is increased owing to the above-described treatments, and is preferably at least 20%, more preferably at least 30%, and even more preferably at least 40%. Furthermore, in the method of the present invention, when the concentration is performed before or after the treatment with the salt and/or alkaloid, this ratio is preferably at least 35%, more preferably at least 40%, even more preferably 45% to 95%, and most preferably 50% to 80%. The proanthocyanidin-containing product obtained by performing the treatment with the salt and/or alkaloid and the synthetic resin adsorbent treatment after the concentration of the extract or the like has a higher OPC ratio in the total proanthocyanidins than the extract or the like, or a treated product obtained by performing only the treatment with the salt and/or alkaloid or the synthetic resin adsorbent treatment without performing the concentration. The amount of OPCs is preferably at least twice, more preferably 2 to 5 times, even more preferably 2 to 4 times. The higher the ratio of the OPCs in the total proanthocyanidins in the proanthocyanidin-containing product is, the higher the bioactivity and the solubility in water of the product.

The obtained proanthocyanidin-containing product further may contain catechins preferably at 10 to 15 wt% in dry weight. Catechins have poor water solubility and exhibit low bioactivities, but water solubility and bioactivities of catechins are increased in the presence of OPCs. Therefore, the proanthocyanidin-containing product containing the OPCs and the catechins are particularly useful.

The term of "Catechins" is a general term referring to polyhydroxyflavan-3-ols. Examples of catechins include (+)-catechin, (-)-epicatechin, (+)-gallocatechin, (-)-epigallocatechin, epigallocatechin gallate, and epicatechin gallate. The catechins also include afzelechin and 3-galloyl derivatives of (+)-catechin or gallocatechin derived from natural products.

The catechins are contained in plants of Family Camellia (e.g., leaves of tea such as green tea, or black tea, oolong tea), Ephedra sinica or the like. For example, the extracts or the like of these plants may be added to the proanthocyanidin-containing product as described above, or a plant extract or the like (e.g., pine bark extract) containing both the proanthocyanidins and the catechins may be used.

Examples of the effects of the catechins include a cancer inhibiting effect, an arteriosclerosis preventing effect, a fat metabolism disorder inhibiting effect, a blood pressure elevation inhibiting effect, a platelet aggregation inhibiting effect, an antiallergic effect, an antiviral effect, an antibacterial effect, a dental caries preventing effect, a halitosis preventing effect, an intestinal flora normalization effect, an active oxygen or free radical eliminating effect, an antioxidation effect and an antidiabetic effect of inhibiting an elevation of blood glucose.

The proanthocyanidin-containing product of the present invention can be utilized in various applications after being concentrated to increase the concentration. For the concentration, various methods such as membrane concentration, heat concentration, vacuum (reduced pressure) concentration, and freeze concentration can be employed.

Furthermore, if necessary, the proanthocyanidin-containing product can be subjected to sterilization treatment for storage. Sterilization can be performed by methods commonly used by those skilled in the art such as stream sterilization, high pressure sterilization, and heat sterilization.

The proanthocyanidin-containing product may be concentrated, dried and powdered after sterilization. Drying can be performed by methods commonly used by those skilled in the art. Among these, freeze drying, vacuum drying and spray drying are preferably used.

The obtained proanthocyanidin-containing product can be utilized as a health drink and a gelled drink and food. Furthermore, the proanthocyanidin-containing product not only can be taken as a drink or food as it is, but also can be mixed with excipients, extenders, binders, thickners, emulsifiers, flavors, food additives, or seasonings, and can be formed into granules, tablets or other forms, depending on the application. For example, the proanthocyanidin-containing product can be mixed with royal jelly, vitamins, protein, calcium, chitosan, lecithin, caffeine, or the like, and its flavor is controlled with sugar solution and seasonings. Furthermore, this product can be formed into capsules such as hard capsules and soft capsules, pills, or tea bag form. This product can be taken as it is, depending on the form or individual preference, or can be dissolved in water, hot water or milk for drinking. In the case of tea bag form, the components obtained by percolation mey be drink.

As described above, the proanthocyanidin-containing product obtained by the present invention can be widely used as a raw material of foods, cosmetics and pharmaceuticals.

### Examples

Hereinafter, the present invention will be described by way of examples. However, the present invention is not limited to these examples. The abbreviation "v/v" shown in the examples refers to "volume/volume", "w/w" refers to "weight/weight" and "w/v" refers to "weight/volume".

First, Reference Examples 1 to 16 were performed as below in order to examine a change in the content of proanthocyanidins by the treatment with a salt or the concentration.

### (Reference Example 1)

First, 7.2 L of purified water was added to 900 g of pine bark, and the pine bark was pulverized with a blender (Waring Blender). The pine bark was extracted with the water under heating at 100°C for 10 minutes. Then, immediately after that, the mixture was filtrated, and the resultant insoluble substances were washed with 1.8 L of purified water. The washing liquid was combined with the filtrated liquid to obtain 9 L of pine bark extract liquid.

When 1 mL of this extract liquid was freeze-dried, the dry weight was 8 mg. Then, 1 L of the extract liquid (the dry weight of the extract powder: 8g) was allowed to cool to 25°C, and sodium chloride was added thereto such that the concentration of the sodium chloride was 35.8 w/v% (i.e., saturated concentration), and the resultant mixture was stirred sufficiently. This mixture was allowed to stand at 4°C for 24 hours, and then filtrated, so that 910 mL of a crude proanthocyanidin-containing liquid A was obtained.

An amount of the proanthocyanidins having a degree of polymerization of 5 or more contained in the obtained crude proanthocyanidin-containing liquid A was compared with that of the pine bark extract before the salt treatment in the following manner.

First, 2-, 4-, 6-, and 8- fold diluted solutions were prepared by diluting 100 µL of the pine bark extract liquid before the salt treatment with purified water. Then, the original pine bark extract liquid and 2-to 8-fold diluted solutions of the pine bark extract liquid and the crude proanthocyanidin-containing liquid A, were subjected to silica gel thin layer chromatography (TLC) to detect proanthocyanidins having a degree of polymerization of 5 or more in each solution.

The elution conditions of TLC and the detection method were as follows:
TLC: silica gel plate manufactured by Merck & Co., Inc.
Eluent: benzene/ethyl formate/formic acid (2/7/1)
Detection reagent: a mixture of sulfuric acid and anisaldehyde
Amount of sample liquid: 10 µL each

After color development with the detection reagent, the degree of the color development of the proanthocyanidins having a degree of polymerization of 5 or more (Rf value : 0.1 or less) contained in the crude proanthocyanidin-containing liquid A was compared with those of the original pine bark extract liquid and 2- to 8-fold diluted solutions of the pine bark extract liquid before the treatment, thereby measuring an approximate amount of the proanthocyanidins having a degree of polymerization of 5 or more in the liquid A. Table 1 shows the results. In Table 1, when the color development of the crude proanthocyanidin-containing liquid A is stronger than that of the original pine bark extract liquid and the diluted solutions, "+" is shown. When it is weaker, "-" is shown. As recognized from Table 1, the color development of the crude proanthocyanidin-containing liquid A is weaker than that of the 6-fold diluted solution of the original pine bark extract liquid, and is stronger than that of the 8-fold diluted solution. Therefore, the content of the proanthocyanidins having a degree of polymerization of 5 or more in the liquid A is considered to be between 1/6 to 1/8 of the original liquid.

### (Reference Example 2)

First, 900 mL of a crude proanthocyanidin-containing liquid B was obtained in the same manner as in Reference Example 1, except that sodium chloride was added so that the concentration of sodium chloride was 25.8 w/v% (70% saturated concentration). Then, an approximate amount of the proanthocyanidins having a degree of polymerization of 5 or more in the liquid B was measured by TLC. Table 1 also shows the results.

### (Reference Example 3)

First, 900 mL of a crude proanthocyanidin-containing liquid C was obtained in the same manner as in Reference Example 1, except that sodium chloride was added so that the concentration of sodium chloride was 17.9 w/v% (50% saturated concentration). Then, an approximate amount of the proanthocyanidins having a degree of polymerization of 5 or more in the liquid C was measured by TLC. Table 1 also shows the results.

### (Reference Example 4)

First, 920 mL of a crude proanthocyanidin-containing liquid D was obtained in the same manner as in Reference Example 1, except that sodium chloride was added so that the concentration of sodium chloride was 10.7 w/v% (30% saturated concentration). Then, an approximate amount of the proanthocyanidins having a degree of polymerization of 5 or more in the liquid D was measured by TLC. Table 1 also shows the results.

### (Reference Example 5)

First, 900 mL of a crude proanthocyanidin-containing liquid E was obtained in the same manner as in Reference Example 1, except that ammonium sulfate was added instead of sodium chloride so that the concentration of ammonium sulfate was 76.8 w/v% (saturated concentration). Then, an approximate amount of the proanthocyanidins having a degree of polymerization of 5 or more in the liquid E was measured by TLC. Table 1 also shows the results.

### (Reference Example 6)

First, 900 mL of a crude proanthocyanidin-containing liquid F was obtained in the same manner as in Reference Example 1, except that ammonium sulfate was added instead of sodium chloride so that the concentration of ammonium sulfate was 53.8 w/v% (70% saturated concentration). Then, an approximate amount of the proanthocyanidins having a degree of polymerization of 5 or more in the liquid F was measured by TLC. Table 1 also shows the results.

### (Reference Example 7)

First, 900 mL of a crude proanthocyanidin-containing liquid G was obtained in the same manner as in Reference Example 1, except that sodium chloride was added so that the concentration of sodium chloride was 1.8 w/v% (5% saturated concentration). Then, an approximate amount of the proanthocyanidins having a degree of polymerization of 5 or more in the liquid G was measured by TLC. Table 1 also shows the results.

### (Reference Example 8)

First, 900 mL of a crude proanthocyanidin-containing liquid H was obtained in the same manner as in Reference Example 1, except that ammonium sulfate was added instead of sodium chloride so that the concentration of ammonium sulfate was 7.7 w/v% (10% saturated concentration). Then, an approximate amount of the proanthocyanidins having a degree of polymerization of 5 or more in the liquid H was measured by TLC. Table 1 also shows the results.

### (Reference Example 9)

First, 7.2 L of 80 v/v% ethanol aqueous solution was added to 900 g of pine bark, and the pine bark was pulverized with a blender (Waring Blender). The pine bark was extracted with the ethanol aqueous solution under heating at 70°C for 1 hour while being refluxed. Then, immediately after that, the mixture was filtrated, and the resultant insoluble substances were washed with 1.8 L of 80 v/v% ethanol aqueous solution. The washing liquid was combined with the filtrated liquid to obtain 9 L of aqueous ethanol extract liquid of pine bark.

When 10 mL of this extract liquid was freeze-dried, the dry weight was 100 mg. Then, 1 L of the extract liquid (the dry weight of the extract powder: 10g) was allowed to cool to 25°C, and concentrated to remove the ethanol completely. Thereafter, purified water was added to adjust the volume to 50 mL, and thus a concentrate liquid I having a volume of 1/20 compared with that of the extract liquid was obtained. Then, 9 g of sodium chloride was added to 50 mL of the obtained concentrate liquid I, and the mixture was stirred sufficiently (the concentration of the sodium chloride was about 17.9 w/v%; about 50% saturated concentration). The resultant solution was allowed to stand at 4°C for 24 hours, and then the precipitated insoluble substances were removed by filtration, so that 52 mL of a crude proanthocyanidin-containing liquid I1 was obtained.

An approximate amount of the proanthocyanidins having a degree of polymerization of 5 or more in the obtained crude proanthocyanidin-containing liquid I1 was measured by the use of TLC in the same manner as in Reference Example 1. Table 1 shows the results.

### (Reference Example 10)

First, 3.6 g of sodium chloride was added to 50 mL of the concentrate liquid I obtained in Reference Example 9, and the mixture was stirred sufficiently (the concentration of the sodium chloride was about 7.2 w/v%, about 20% saturated concentration). The resultant solution was allowed to stand at 4°C for 24 hours, and then the precipitated insoluble substances were removed by filtration, so that 51 mL of a crude proanthocyanidin-containing liquid I2 was obtained. Based on the amount of the proanthocyanidins having a degree of polymerization of 5 or more in the solution (concentrate liquid I) before the salt treatment, an approximate amount of the proanthocyanidins having a degree of polymerization of 5 or more in the liquid I2 was measured by the use of TLC in the same manner as in Reference Example 1. Table 1 shows the results.

### (Reference Example 11)

First, 19.2 g of ammonium sulfate was added to 50 mL of the concentrate liquid I obtained in Reference Example 9, and the mixture was stirred sufficiently (the concentration of the ammonium sulfate was about 38.4 w/v°/, about 50% saturated concentration). The resultant solution was allowed to stand at 4°C for 24 hours, and then the precipitated insoluble substances were removed by filtration, so that 51 mL of a crude proanthocyanidin-containing liquid I3 was obtained. Based on the amount of the proanthocyanidins having a degree of polymerization of 5 or more in the solution (concentrate liquid I) before the salt treatment, an approximate amount of the proanthocyanidins having a degree of polymerization of 5 or more in the liquid 13 was measured by the use of TLC in the same manner as in Reference Example 1. Table 1 shows the results.

### (Reference Example 12)

The ethanol extract liquid of pine bark was concentrated in the same manner as in Reference Example 9, and the volume was adjusted to 100 mL with purified water. Thus, a concentrate liquid J having a volume of 1/10 compared with that of the extract liquid was obtained. Then, 17.9 g of sodium chloride was added to 100 mL of this concentrate liquid J, and the mixture was stirred sufficiently (the concentration of the sodium chloride was about 17.9 w/v%, about 50% saturated concentration). The resultant solution was allowed to stand at 4°C for 24 hours, and then the precipitated insoluble substances were removed by filtration, so that 96 mL of a crude proanthocyanidin-containing liquid J was obtained. Based on the amount of the proanthocyanidins having a degree of polymerization of 5 or more in the solution (concentrate liquid J) before the salt treatment, an approximate amount of the proanthocyanidins having a degree of polymerization of 5 or more in the liquid J was measured by the use of TLC in the same manner as in Reference Example 1. Table 1 shows the results.

### (Reference Example 13)

First, 7.2 L of purified water was added to 900 g of pine bark, and the pine bark was pulverized with a blender (Waring Blender). The pine bark was extracted with the water under heating at 100°C for 10 minutes. Then, immediately after that, the mixture was filtrated, and the resultant insoluble substances were washed with 1.8 L of purified water. The washing liquid was combined with the filtrated liquid to obtain 9 L of water extract liquid of pine bark.

When 10 mL of this extract liquid was freeze-dried, the dry weight was 73 mg. Then, 1 L of the extract liquid (the dry weight of the extract powder: 7.3 g) was allowed to cool to 25°C. The water extract liquid was applied on a column filled with 500 mL of DIAION HP-20 that was swollen with water. The column was washed with 3 L of purified water, and then all the adsorbed components were eluted with 500 mL of absolute ethanol. The eluate was subjected to vacuum concentration to remove the ethanol completely, and the volume was adjusted to 50 mL with purified water. Thus, a concentrate liquid K having a volume of 1/20 compared with that of the extract liquid was obtained. The treatment with a salt was performed in the same manner as in Reference Example 9, and the precipitated insoluble substances were removed by filtration, so that 51 mL of a crude proanthocyanidin-containing liquid K was obtained. Based on amount of the proanthocyanidins having a degree of polymerization of 5 or more in the the solution (concentrate liquid K) before the treatment with a salt, an approximate amount of the proanthocyanidins having a degree of polymerization of 5 or more in the liquid K was measured by the use of TLC in the same manner as in Reference Example 1. Table 1 shows the results.

### (Reference Example 14)

After the ethanol extract liquid of pine bark was concentrated in the same manner as in Reference Example 9, the volume was adjusted to 1 L with purified water. Thus, a concentrate liquid L having a volume of 1/1 compared with that of the extract liquid was obtained. Then, 179 g of sodium chloride was added to 1 L of this concentrate liquid L, and the mixture was stirred sufficiently (the concentration of the sodium chloride was about 17.9 w/v%; about 50% saturated concentration). The resultant solution was allowed to stand at 4°C for 24 hours, and then the precipitated insoluble substances were removed by filtration, so that 950 mL of a crude proanthocyanidin-containing liquid L1 was obtained. Based on the amount of the proanthocyanidins having a degree of polymerization of 5 or more in the solution (concentrate liquid L) before the salt treatment, an approximate amount of the proanthocyanidins having a degree of polymerization of 5 or more in the liquid L1 was measured by the use of TLC in the same manner as in Reference Example 1. Table 1 shows the results.

### (Reference Example 15)

First, 72 g of sodium chloride was added to 1 L of the concentrate liquid L obtained in Reference Example 14, and the mixture was stirred sufficiently (the concentration of the sodium chloride was about 7.2 w/v%, about 20% saturated concentration). The resultant solution was allowed to stand at 4°C for 24 hours, and then the precipitated insoluble substances were removed by filtration, so that 930 mL of a crude proanthocyanidin-containing liquid L2 was obtained. Based on the amount of the proanthocyanidins having a degree of polymerization of 5 or more in the solution (concentrate liquid L) before the salt treatment, an approximate amount of the proanthocyanidins having a degree of polymerization of 5 or more in the liquid L2 was measured by the use of TLC in the same manner as in Reference Example 1. Table 1 shows the results.

### (Reference Example 16)

First, 384 g of ammonium sulfate was added to 1 L of the concentrate liquid L obtained in Reference Example 14, and the mixture was stirred sufficiently (the concentration of the ammonium sulfate was about 38.4 w/v%, about 50% saturated concentration). The resultant solution was allowed to stand at 4°C for 24 hours, and then the precipitated insoluble substances were removed by filtration, so that 970 mL of a crude proanthocyanidin-containing liquid L3 was obtained. Based on the amount of the proanthocyanidins having a degree of polymerization of 5 or more in the solution (concentrate liquid L) before the salt treatment, an approximate amount of the proanthocyanidins having a degree of polymerization of 5 or more in the liquid L3 was measured by the use of TLC in the same manner as in Reference Example 1. Table 1 shows the results.

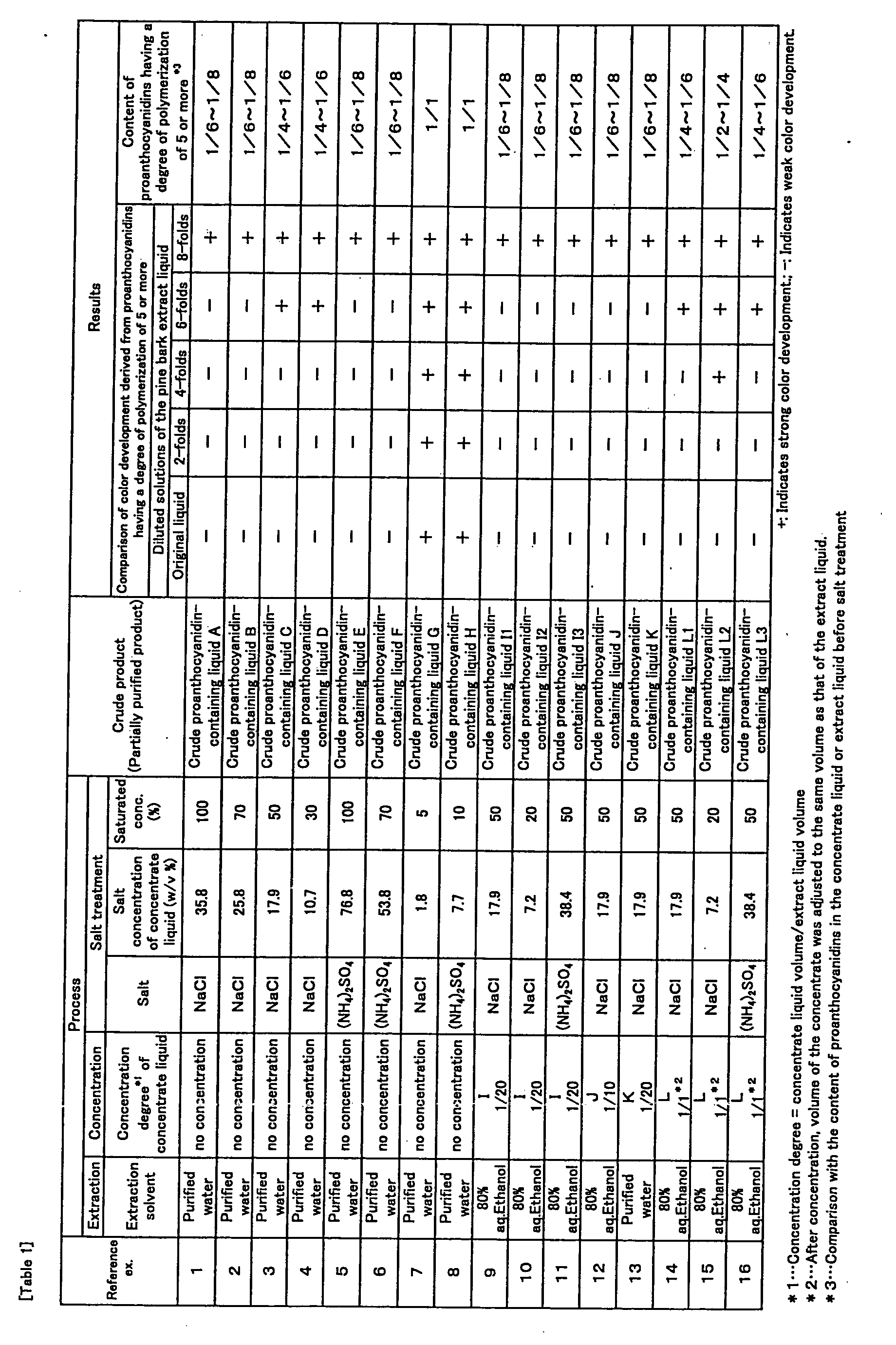

From Table 1, the following results are shown. In the case where the concentration was not performed (Reference Examples 1 to 8), each of the crude proanthocyanidin-containing liquids A and B and the crude proanthocyanidin-containing liquids E and F provided weaker color development than that of the 6-fold diluted liquids of the original pine bark extract liquid, wherein the crude proanthocyanidin-containing liquids A and B were obtained by treating with a sodium chloride solution having a concentration that is 70% or more of the saturated concentration, and the crude proanthocyanidin-containing liquids E and F were obtained by treating with ammonium sulfate having a concentration that is 70% or more of the saturated concentration. On the other hand, in the case where the concentration was performed, each of the crude proanthocyanidin-containing liquids I (I1 to I3), J, and K (Reference Examples 9 to 12) provided weaker color development than that of the 6-fold diluted liquids of the solutions (concentrate liquids) before the salt treatment, wherein each of the crude proanthocyanidin-containing liquids I (I1 to I3), J, and K are obtained by concentrating the extract, and treating the concentrate with a salt, and wherein the concentrate has not been diluted. These facts indicate that in the crude proanthocyanidin-containing liquids A, B, E, F, I (I1 to I3), J and K, the amount of the proanthocyanidins having a degree of polymerization of 5 or more was reduced to 1/6 to 1/8 of that of the original pine bark extract liquid before the salt treatment. In particular, it is indicated that in the case where the concentration was performed, the amount of the salt can be reduced. Hereinafter, the obtained crude proanthocyanidin-containing liquids were subjected to further purification.

### (Example 1)

The crude proanthocyanidin-containing liquid A was further purified in the following manner. First, 100 mL of an aromatic synthetic resin (DIAION HP-20 manufactured by Mitsubishi Chemical Corporation) swollen with water were filled in a 30 x 300mm column. Then, 900 mL of the crude proanthocyanidin-containing liquid A was applied on the above-described column, so that the proanthocyanidins were adsorbed to the resin in the column. This column was washed with 1 L of purified water to remove saccharides, organic acids and the like that remained in the column. Next, the proanthocyanidins were eluted from the column, using a 15 v/v% ethanol-water mixed solvent, and thus 200 mL of purified proanthocyanidin-containing liquid A was obtained. This proanthocyanidin-containing liquid A was freeze-dried and its dry weight was measured.

Next, in order to measure the content of each component of the obtained purified proanthocyanidin-containing liquid A, it was separated into a fraction containing OPCs, a fraction of proanthocyanidins having a degree of polymerization of 5 or more, a fraction containing catechins, and a fraction containing other components than catechins in the following manner. First, 25 mL of Sephadex LH-20 (manufactured by Amersham Biotech) swollen with water were filled in a 15 x 300 mm column, and washed with 50 mL of ethanol. Next, 100 mg of the above-described dry powder were dissolved in 2 mL of ethanol, and this solution was applied on the column so that proanthocyanidins were adsorbed. Then, gradient elution was conducted using 100 to 80 v/v% ethanol-water mixed solvents, and the resultant eluate was collected in fractions of 10 mL each. Each of the collected fractions was immediately subjected to TLC in the same manner as in Reference Example 1 to detect OPCs using specimens of dimeric to tetrameric OPCs (dimer: proanthocyanidin B-2 (Rf value: 0.6), a trimer: proanthocyanidin C-1 (Rf value: 0.4), and a tetramer: cinnamtannin A₂ (Rf value: 0.2)) as indicators.

The eluted fractions that were confirmed to contain OPCs by TLC were combined so that an OPC fraction was obtained.

Then, at the point when the OPCs were not detected any more, 300 mL of 50 v/v% water-acetone mixed solvent was allowed to flow through the column so that the remaining adsorbed substances that were adsorbed to the column were eluted.

The collected fractions containing the remaining adsorbed substances were combined, and separated into a fraction containing catechins and a fraction of proanthocyanidins having a degree of polymerization of 5 or more by TLC, using catechin (Rf value: 0.8) as an indicator. The developing conditions of TLC and the detection method were the same as above.

The fraction containing catechins was further separated into the catechins and other components than the catechins in the following manner. First, the fraction containing catechins was freeze-dried so that powder was obtained. This powder was dissolved in 3 mL of water, and this solution was applied on a 15 x 300 mm column filled with 20 mL of MCI Gel (manufactured by Mitsubishi Chemical Corporation) swollen with water for adsorption. This column was washed with water, and then gradient elution was conducted using 10 v/v°/ to 100 v/v% ethanol-water mixed solvents, and the resultant eluate was collected in fractions of 7 mL each. After the elution, the catechins in each fraction were detected by TLC using the catechin as an indicator, so that the fraction was separated into a catechin fraction and a fraction of other components than the catechins.

The thus obtained OPC fraction, a fraction of proanthocyanidins having a degree of polymerization of 5 or more, catechin fraction, and fraction of other components than the catechins were powdered by freeze-drying and the dry weight was measured. The total of the OPC fraction, the fraction of proanthocyanidins having a degree of polymerization of 5 or more, the catechin fraction, the fraction of other components than the catechins and a fraction of other components was 99.2 mg to 99.6 mg with respect to 100 mg of the dry powder of the purified proanthocyanidin-containing liquid A. This means that almost all the components had been recovered.

Table 2 shows the dry weight of the purified proanthocyanidin-containing liquid A, the dry weights and contents of the OPCs, the proanthocyanidins having a degree of polymerization of 5 or more, the total of the proanthocyanidins (total of the OPCs and the proanthocyanidins having a degree of polymerization of 5 or more), and the catechins contained in the purified proanthocyanidin-containing liquid A. Table 2 also shows the ratio of the OPCs in the total of the proanthocyanidins. In Table 2, "a" shows the weight of the solid, "b" shows the OPC weight, "c" shows the weight of the proanthocyanidins having a degree of polymerization of 5 or more, and "d" shows the weight of the catechins.

### (Examples 2 to 8)

From the crude proanthocyanidin-containing liquids B to H, purified proanthocyanidin-containing liquids B to H were obtained in the manner as in Example 1, respectively. Then, the dry weight of each of the purified proanthocyanidin-containing liquids B to H was measured. Furthermore, the amount of the OPCs, the amount of the proanthocyanidins having a degree of polymerization of 5 or more, the total amount of the proanthocyanidins, and the amount of catechins contained in the purified proanthocyanidin-containing liquids B to G, and also, the ratio of the OPCs in the total of the proanthocyanidins were measured. Table 2 shows the results.

### (Examples 9 to 14)

From the crude proanthocyanidin-containing liquids I1 to I3, J, K and L1, purified proanthocyanidin-containing liquids I1 to I3, J, K and L1 were obtained, respectively, in the same manner as in Example 1, except that the 15 v/v% ethanol-water mixed solution as the eluent was replaced by 20 v/v% ethanol-water mixed solution, and were measured regarding each item shown in Table 2. Table 2 also shows the results.

### (Example 15)

First, 7.2 L of 80 v/v% ethanol aqueous solution was added to 900 g of pine bark, and the pine bark was pulverized with a blender (Waring Blender). The pine bark was extracted with the ethanol aqueous solution under heating at 100°C for 1 hour while being refluxed. Then, immediately after that, the mixture was filtrated, and the resultant insoluble substances after the filtration were washed with 1.8 L of 80 v/v% ethanol aqueous solution. The washing liquid was combined with the filtrated liquid to obtain 7.6 L of aqueous ethanol extract liquid of pine bark.

Then, 1 L of the extract liquid was allowed to cool to 25°C, and concentrated by vacuum concentrations while the ethanol was completely removed. Thereafter, the volume was adjusted to 50 mL with purified water. Thus, a concentrate liquid M having a volume of 1/20 compared with that of the extract liquid was obtained. Then, 0.005 g of caffeine was added to 50 mL of the obtained concentrate liquid M, and the mixture was stirred sufficiently (the concentration of the caffeine was about 0.01 w/v%). The resultant solution was allowed to stand at 4°C for 24 hours, a crude proanthocyanidin-containing liquid M was obtained.

From 900 mL of the crude proanthocyanidin-containing liquid M, a purified proanthocyanidin-containing liquid M was obtained by performing purification in the same manner as in Example 1, except that the 15 v/v% ethanol-water mixed solution as the eluent was replaced by 20 v/v% ethanol-water mixed solution, and was measured regarding each item shown in Table 2. Table 2 also shows the results.

### (Example 16)

A purified proanthocyanidin-containing liquid N was obtained by performing purification in the same manner as in Example 15, except that betaine was added in an amount of 0.005 g instead of caffeine. The concentration of betaine was about 0.01 w/v%. The purified proanthocyanidin-containing liquid N was measured regarding each item shown in Table 2. Table 2 also shows the results.

### (Example 17)

The aqueous ethanol extract liquid of pine bark obtained in Reference Example 9 was concentrated to 1/20 volume to prepare a concentrate liquid I. Then, 0.025 g of magnesium sulfate was added to the concentrate liquid I (50mL), and the mixture was stirred sufficiently. The concentration of the magnesium sulfate was about 0.05 w/v%. The resultant solution was allowed to stand at 4°C for 24 hours, and thus a crude proanthocyanidin-containing liquid I4 was obtained. Then, 900 mL of the crude proanthocyanidin-containing liquid I4 was purified in the same manner as in Example 1, so that a purified proanthocyanidin-containing liquid 14 was obtained). The purified proanthocyanidin-containing liquid 14 was measured regarding each item shown in Table 2. Table 2 also shows the results.

### (Comparative Example 1)

Purification was performed in the same manner as in Example 1, except that the pine bark extract liquid (purified water extract) obtained in Reference Example 1 was used, instead of the crude proanthocyanidin-containing liquid A, and measurement was performed, regarding each item shown in Table 2. Table 2 also shows the results.

### (Comparative Example 2)

Purification was performed in the same manner as in Example 1, except that the aqueous ethanol extract liquid of pine bark obtained in Reference Example 9 was used, instead of the crude proanthocyanidin-containing liquid A, and measurement was performed, regarding each item shown in Table 2. Table 2 also shows the results.

### (Comparative Example 3)

A betaine extract liquid of pine bark was obtained in the same manner as in Example 1, except that a solution (betaine-containing solution) obtained by adding 0.005 g of betaine to 1 L of 80 v/v% ethanol aqueous solution was used, instead of the purified water. Furthermore, purification was performed in the same manner as in Example 1, except that the above betaine extract liquid of pine bark was used, instead of the crude proanthocyanidin-containing liquid A, and measurement was performed, regarding each item shown in Table 2. Table 2 also shows the results.

### (Comparative Example 4)

In the process of adding magnesium sulfate to the concentrate liquid I (50mL) of Example 17, 0.2 g of ascorbic acid was added. Furthermore, 0.2 g of magnesium sulfate was added, and then, 0.2 g of calcium hydroxide was added, and the resultant insoluble components were recovered. These insoluble substances were dissolved in 50 mL of a solution containing 3 v/v% of hydrochloric acid, and purification was performed in the same manner as in Example 1. Then, measurement was performed, regarding each item shown in Table 2. Table 2 also shows the results.

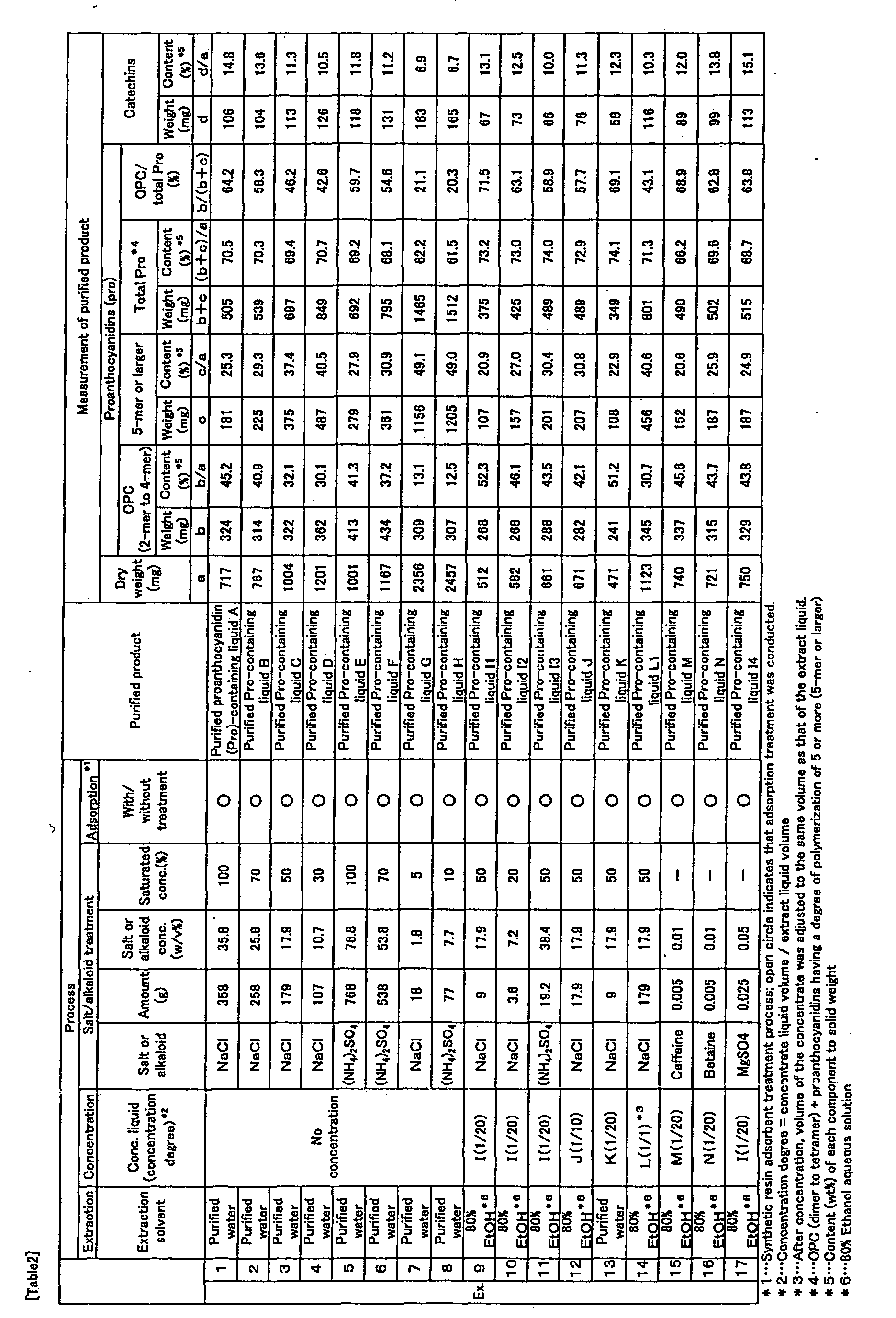

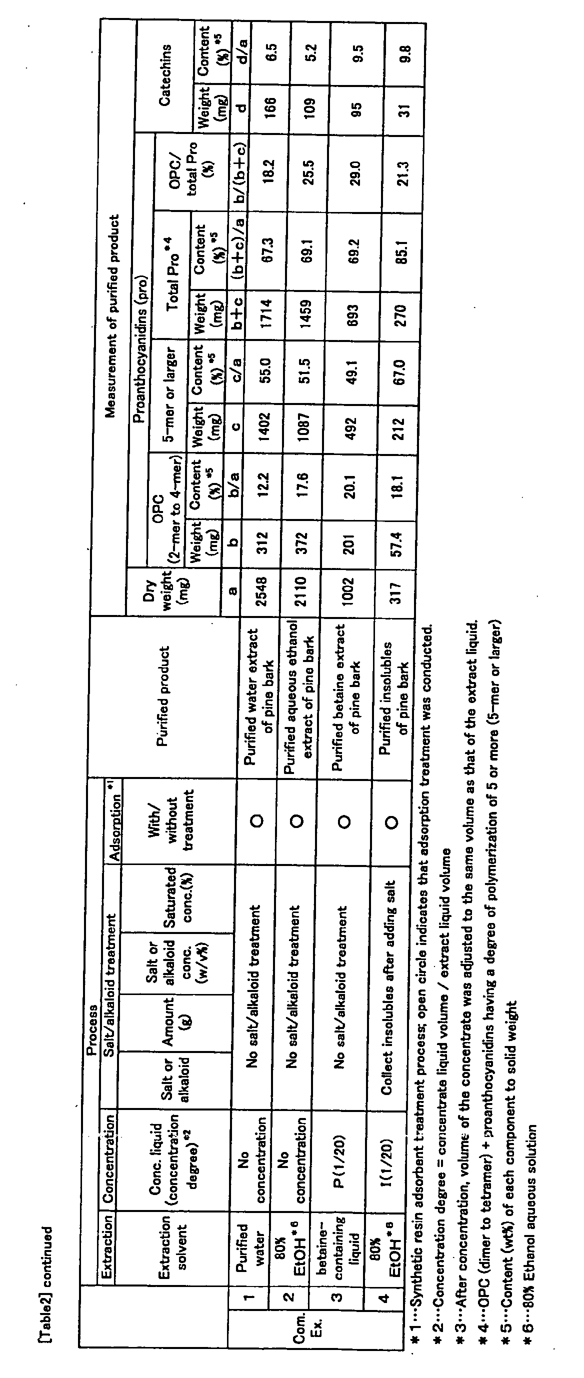

As seen from the results of Table 2, the purified proanthocyanidin-containing liquids that had been subjected to the salt and/or alkaloid treatment and the synthetic resin adsorbent treatment contained proanthocyanidins at a high ratio. The purified proanthocyanidin-containing liquids A to F (Examples 1 to 6) that were obtained with the salt treatment and without the concentration process had a particularly high content of OPCs, which was 30 wt% or more in terms of dry weight. In other words, the proanthocyanidin-containing liquid that was obtained by adding sodium chloride or ammonium sulfate to a pine bark extract liquid such that the final concentration was 30% or more based on the saturated concentration contained OPCs and catechins at a high concentration. Furthermore, for the purified proanthocyanidin-containing liquids A to F of Examples 1 to 6, although the dry weight of the proanthocyanidins having a degree of polymerization of 5 or more is reduced compared with that of the purified proanthocyanidin-containing liquids G and H of Examples 7 and 8, there is no large difference in the dry weight of the OPCs, so that the ratio of the OPCs in the total proanthocyanidins is increased remarkably. In particular, the amount of OPCs is 2 to 3.5 times as large as the amount of the untreated pine bark extract of Comparative Example 1. From the above, it was confirmed that by adding a predetermined amount of a monovalent alkali metal salt or ammonium sulfate, a proanthocyanidin-containing product containing a large amount of OPCs. can be obtained from a plant without adding, for example, an aid for stabilizing proanthocyanidins.

All of the purified proanthocyanidin-containing liquids I (I1 to I4), J, K, M, and N obtained through the salt and/or alkaloid treatment conducted after the concentration process contained OPCs in an amount of 35 wt% or more in terms of dry weight (Examples 9 to 13 and 15 to 17).

The purified proanthocyanidin-containing liquids I1 to I3, J and K of Examples 9 to 13 were obtained using a small amount of the salt in the salt treatment, which is an amount of 1/9 to 1/20 compared with that used for obtaining the purified proanthocyanidin-containing liquid C of Example 3 and that used for obtaining the purified proanthocyanidin-containing liquid L1 of Example 14, wherein the purified proanthocyanidin-containing liquid C was obtained by subjecting the extract liquid directly to the salt treatment, and the purified proanthocyanidin-containing liquid L1 was obtained by concentrating the extract liquid, diluting it to the original concentration, and subjecting it to the salt treatment. However, they contained OPCs at a high concentration, and thus they are excellent in respect to the cost and environmental safety. In addition, the content of catechins was increased. Furthermore, in the purified proanthocyanidin-containing liquids I1 to I3, J and K of Examples 9 to 13, the contents (weight) of proanthocyanidins having a degree of polymerization of 5 or more are lower, but the OPC contents (weight) are not so lower than in the purified pine bark extract liquids and purified pine bark insoluble substance of Comparative Examples 1 to 4. Therefore, the ratio of the OPCs in the total proanthocyanidins is as remarkably high as 57 wt% to 71.5 wt%. In particular, the content of the OPCs in solid substances is as remarkably high as 2.3 times to 2.9 times and the content of the OPCs to the total proanthocyanidins is as remarkably high as 2.2 times to 2.8 times the content of the OPCs in the aqueous ethanol extract liquid of pine bark of Comparative Example 2 that has not been concentrated and has not been subjected to the salt treatment. For the purified proanthocyanidin-containing liquids I1 to I3, J and K of Examples 9 to 13, the content of the OPCs in the solid is higher, which is 1.3 times to 1.7 times and the content of the OPCs to the total proanthocyanidins is higher, which is 1.3 times to 1.7 times the content of the purified proanthocyanidin-containing liquid C of Example 3 and the content of the purified proanthocyanidin-containing liquid L1 of Example 14, wherein the purified proanthocyanidin-containing liquid C was obtained by subjecting the extract liquid directly to the salt treatment, and the purified proanthocyanidin-containing liquid L1 was obtained by concentrating the extract liquid, diluting it to the original concentration, and subjecting it to the salt treatment. From the above, it was confirmed that a proanthocyanidin-containing product containing a large amount of OPCs can be obtained from a plant by subjecting a pine bark extract liquid to the concentration process, the salt and/or alkaloid treatment, and the synthetic resin adsorbent treatment. In the salt treatment, the amount of the salt used is small.

The purified proanthocyanidin-containing liquids M, N and I4 of Examples 15 to 17 obtained by using an alkaloid and a divalent metal salt have a high OPC content and contain less proanthocyanidins having a degree of polymerization of 5 or more compared with the extract liquid obtained by the use of a alkaloid (i.e., betaine)-containing solution (Comparative Example 3) and the insoluble substances (Comparative Example 4) that were formed by the addition of a divalent metal salt (magnesium sulfate or calcium hydroxide). In particular, this is prominent when caffeine or a divalent metal salt is used. The amount of the alkaloid or the salt used is quite small compared with the case where a monovalent metal salt or ammonium sulfate, which is economically advantageous and advantageous from the viewpoint of liquid waste disposal.

### (Example 18)

First, 1 L of purified water was added to 100 g of pine bark and the pine bark was pulverized and extraction was performed with heating at 100°C for 10 minutes. Then, immediately after that, the mixture was filtrated, and the resultant insoluble substance after the filtration was washed with 200 mL of purified water. The washing liquid was combined with the filtrated liquid to obtain 1.2 L of an extract liquid. A part of this extract liquid (5 mL) was subjected to vacuum concentration, and the weight of the obtained extract powder was measured (39 mg).

Then, the extract powder was added to the remaining extract liquid, and the mixture was adjusted with hot water such that its total amount was 1.2L. Then, the mixture was allowed to cool to 25°C, 2.4 L of an aqueous solution (pH5: adjusted with citric acid) containing 30 wt% of calcium chloride was added thereto. The resultant mixture was stirred, and then was allowed to stand at 4°C for 24 hours. This mixture was filtrated so that a crude proanthocyanidin-containing liquid O was obtained. The crude proanthocyanidin-containing liquid O was adjusted with acetic acid so that the pH was 3. Then, 30 g of DIAION HP-20 was added to this solution, and the mixture was stirred for 3 hours. Thereafter, the mixture was filtrated, and DIAION HP-20(a solid substance) to which proanthocyanidins are adsorbed was obtained. This solid substance was washed with 250 mL of purified water, and 150 mL of 20 v/v% ethanol aqueous solution was added and the resultant mixture was stirred for one hour, and then filtrated. Thereafter, the filtrated liquid was obtained and taken as a purified proanthocyanidin-containing liquid O. This purified proanthocyanidin-containing liquid O was evaporated to dryness under reduced pressure, giving 0.2 g of proanthocyanidin-containing dry powder. Using 100 mg of the proanthocyanidin-containing dry powder, the contents of the OPCs and catechins were measured in the same manner as in Example 1. OPCs were contained in an amount of 48.1 wt%, and catechins were contained in an amount of 15.2 wt% in terms of dry weight.

### (Example 19)

First, 1 L of purified water was added to 100 g of pine bark and the pine bark was pulverized and extraction was performed with heating at 100°C for 10 minutes. Then, immediately after that, the mixture was filtrated, and the resultant insoluble substance after the filtration was washed with 200 mL of purified water. The washing liquid was combined with the filtrated liquid to obtain 1.2 L of an extract liquid including the filtrated liquid. A part of this extract liquid (5 mL) was subjected to vacuum concentration, and the weight of the obtained extract powder was measured (38 mg).

Then, the extract powder was added to the remaining extract liquid, and the mixture was adjusted with hot water such that its total amount was 1.2L. Then, the mixture was allowed to cool to 25°C, 40 g of DIAION HP-20 was added to this solution, and the mixture was stirred for 3 hours. Thereafter, the mixture was filtrated, and DIAION HP-20 (a solid substance) to which proanthocyanidins were adsorbed was obtained. This solid substance was washed with 250 mL of purified water, and 150 mL of 20 v/v% ethanol aqueous solution was added and the resultant mixture was stirred for one hour. The mixture was filtrated, and the filtrated liquid was obtained. Then, sodium chloride was added so that the sodium chloride was contained at the saturated concentration (about 35.8 w/v%). The resultant mixture was stirred, and then was allowed to stand at 4°C for 24 hours. The resultant mixture was filtrated so that a proanthocyanidin-containing liquid P was obtained. The proanthocyanidin-containing liquid P was dialyzed for desalting, and substituted with water to remove salts. The proanthocyanidin-containing liquid P was evaporated to dryness under reduced pressure, giving 1.3 g of proanthocyanidin-containing dry powder. Using 100 mg of the proanthocyanidin-containing dry powder P, the contents of the OPCs and catechins were measured in the same manner as in Example 1. OPCs were contained in an amount of 40.1 wt%, and catechins were contained in an amount of 13.1 wt% in terms of dry weight.

### (Example 20)

First, 1 L of 80 v/v% ethanol aqueous solution was added to 100 g of pine bark, and the pine bark was pulverized with a blender. The pine bark was extracted with the ethanol aqueous solution under heating at 80°C for 1 hour. Then, immediately after that, the mixture was filtrated, and the resultant insoluble substance after the filtration was washed with 200 mL of purified water. The washing liquid was combined with the filtrated liquid to obtain 1.2 L of an extract liquid including the filtrated liquid. A part of this extract liquid (5 mL) was subjected to vacuum concentration, and the weight of the obtained extract powder was measured (49 mg).

Then, the extract powder was added to the remaining extract liquid, and the solution was subjected to vacuum concentration to remove ethanol completely. Thereafter, purified water was added to adjust such that the volume was 100 mL, and then the solution was allowed to cool to 25°C, and thus a concentrate liquid corresponding to 1/12 volume of the extract liquid was obtained. Then, sodium chloride was added to 100 mL of the obtained concentrate liquid so that the sodium chloride was contained at the saturated concentration (about 35.8 w/v%), and the resultant mixture was stirred sufficiently. The mixture was allowed to stand at 4°C for 24 hours, and filtrated so that a crude proanthocyanidin-containing liquid Q was obtained. Then, 900 mL of purified water and 10 g of DIAION HP-20 were added to the crude proanthocyanidin-containing liquid Q, and the mixture was stirred for 3 hours. Thereafter, the mixture was filtrated, and DIAION HP-20 (a solid substance) to which proanthocyanidins were adsorbed was obtained. This solid substance was washed with 250 mL of purified water, and 150 mL of 20 v/v% ethanol aqueous solution was added thereto and the resultant mixture was stirred for one hour. Then, the mixture was filtrated, and the filtrated liquid was collected and taken as a purified proanthocyanidin-containing liquid Q. The purified proanthocyanidin-containing liquid Q was evaporated to dryness under reduced pressure, giving 0.2 g of proanthocyanidin-containing dry powder. Using 100 mg of the proanthocyanidin-containing dry powder, the contents of the OPCs and catechins were measured in the same manner as in Example 1. OPCs were contained in an amount of 51.2 wt%, and catechins were contained in an amount of 13.1 wt% in terms of dry weight.

### (Example 21)

First, 1 L of purified water was added to 100 g of pine bark, , and the pine bark was pulverized with a blender. The pine bark was extracted with the water under heating at 100°C for 10 minutes. Then, immediately after that, the mixture was filtrated, and the resultant insoluble substance after the filtration was washed with 200 mL of purified water. The washing liquid was combined with the filtrated liquid to obtain 1.2 L of an extract liquid. A part of this extract liquid (5 mL) was subjected to vacuum concentration, and the weight of the obtained extract powder was measured (36 mg).

Then, the extract powder was added to the remaining extract liquid, and the mixture was subjected to vacuum concentration. Thereafter, purified water was added to adjust such that the volume became 100 mL. Then, the solution was allowed to cool to 25°C, and thus a concentrate liquid corresponding to 1/12 volume of the extract liquid was obtained. Then, 100 mL of an aqueous solution (pH5: adjusted with citric acid) containing 50 wt% of calcium chloride was added to 100 mL of the obtained concentrate liquid, and the resultant mixture was stirred. This mixture was allowed to stand at 4°C for 24 hours, and then was filtrated, so that a crude proanthocyanidin-containing liquid R was obtained. Acetic acid and purified water were added to the crude proanthocyanidin-containing liquid R to prepare 1 L of a solution with pH 3. Then, 10 g of DIAION HP-20 was added to this solution, and the mixture was stirred for 3 hours. Thereafter, the mixture was filtrated, and DIAION HP-20 (a solid substance) to which proanthocyanidins are adsorbed was obtained. This solid substance was washed with 250 mL of purified water, and 150 mL of 20 v/v% ethanol aqueous solution was added. The resultant mixture was stirred for one hour, and filtrated to obtain filtrated liquid, which was taken as a purified proanthocyanidin-containing liquid R. This purified proanthocyanidin-containing liquid R was evaporated to dryness under reduced pressure, giving 0.3 g of proanthocyanidin-containing dry powder R was obtained. Using 100 mg of the proanthocyanidin-containing dry powder R, the contents of OPCs and catechins were measured in the same manner as in Example 1. OPCs were contained in an amount of 46.2 wt%, and catechins were contained in an amount of 10.1 wt% in terms of dry weight.

### Industrial Applicability

According to the method of the present invention, an extract or squeezed juice of a plant is treated with a salt and/or an alkaloid and then treated with a synthetic resin adsorbent. Alternatively, an extract or squeezed juice of a plant is treated with a synthetic resin adsorbent and then treated with a salt and/or an alkaloid. With this, a proanthocyanidin-containing product containing highly bioactive OPCs at a high ratio can be obtained easily and efficiently. This method is particularly useful from the viewpoint of the cost and environmental safety. The obtained proanthocyanidin-containing product having a high OPC content also contains catechins. Therefore, the synergistic effect that the OPCs are activated by the actions of the catechins is provided. The proanthocyanidin-containing products are effective for improving vascular proliferation, hypertension, oversensitiveness to the cold and the like, and are very useful as a raw material for producing foods, cosmetics, pharmaceuticals and the like.

## Claims

1. A method for producing a proanthocyanindin-containing product obtained from an extract or squeezed juice of a plant comprising,
subjecting an extract or squeezed juice of a plant to a combination of the following treatments:
a treatment with at least one of a salt and an alkaloid, and
a treatment with a synthetic resin adsorbent.

2. The method of claim 1, further comprising a concentration process that is conducted before and/or after the treatment with at least one of a salt and an alkaloid, wherein the concentration process excludes the treatment with a synthetic resin adsorbent.

3. A method for producing a proanthocyanidin-containing product comprising the steps of:
subjecting an extract or squeezed juice of a plant to a concentration process to obtain a concentrate;
treating the concentrate with at least one of a salt and an alkaloid to obtain a crude proanthocyanidin-containing product; and
treating the crude proanthocyanidin-containing product with a synthetic resin adsorbent.

4. The method of any one of claims 1 to 3, wherein the salt is at least one selected from the group consisting of a monovalent alkali metal salt, a divalent alkali metal salt, and ammonium sulfate.

5. The method of any one of claims 1 to 3, wherein the alkaloid is at least one selected from the group consisting of betaine, caffeine and their derivatives.

6. The method for producing a proanthocyanidin-containing product of any one of claims 1 to 5, wherein the proanthocyanidin-containing product comprises at least 20 wt% of oligomeric proanthocyanidins in terms of dry weight.

7. A proanthocyanidin-containing product obtained by the method of any one of claims 1 to 6, wherein the proanthocyanidin-containing product comprises at least 20 wt% of oligomeric proanthocyanidins in terms of dry weight.

8. The proanthocyanidin-containing product of claim 7, further comprising 5 to 15 wt% of catechins in terms of dry weight.
